(19) Europäisches Patentamt
European Patent Office
Office européen des brevets

(11) Numéro de publication: **0 050 543**
**B1**

(12) # FASCICULE DE BREVET EUROPEEN

(45) Date de publication du fascicule du brevet: **16.01.85**

(51) Int. Cl.[4]: **C 07 C 69/74**

(21) Numéro de dépôt: **81401521.0**

(22) Date de dépôt: **02.10.81**

(54) **Nouveaux esters terbutyliques dérivés de l'acide 2,2-diméthyl cyclopropane 1,3-dicarboxylique, leur préparation et les nouveaux intermédiaires obtenus.**

(30) Priorité: **10.10.80 FR 8021691**

(43) Date de publication de la demande: **28.04.82 Bulletin 82/17**

(45) Mention de la délivrance du brevet: **16.01.85 Bulletin 85/3**

(84) Etats contractants désignés: **AT BE CH DE GB IT LI LU NL SE**

(56) Documents cités:
**FR - A - 2 376 120**

**CHEMICAL ABSTRACTS, vol. 73, no. 21, 23 novembre 1970 réf. 109322q, page 331 COLUMBUS, OHIO (US)**

(73) Titulaire: **ROUSSEL-UCLAF, 35, boulevard des Invalides, F-75007 Paris (FR)**

(72) Inventeur: **Krief, Alain, 16, rue Tienne aux Clochers, B-5150 Wepion (BE)**

(74) Mandataire: **Douetteau, Pierre et al, ROUSSEL-UCLAF 111, route de Noisy Boîte postale no. 9, F-93230 Romainville (FR)**

**EP 0 050 543 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux esters terbutyliques dérivés de l'acide 2,2-diméthyl cyclopropane-1,3-dicarboxylique, leur préparation ainsi que les nouveaux intermédiaires obtenus.

L'invention a ainsi pour objet les composés répondant à la formule (I)

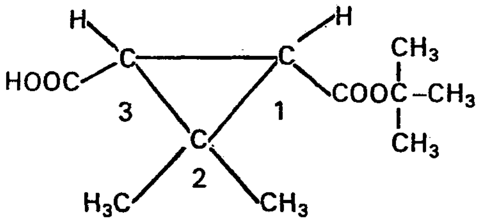

(I)

de configuration cis ou trans, sous leurs formes optiquement actives.

L'invention a donc plus particulièrement pour objet les monoesters terbutyliques des acides (1R,3S) et (1S,3R) cis 2,2-diméthyl cyclopropane 1,3-dicarboxyliques, ainsi que les mono esters terbutyliques des acides (1R,3R) et (1S,3S) trans 2,2-diméthyl cyclopropane 1,3-dicarboxyliques.

L'invention a également pour objet un procédé de préparation des composés de formule (I), telle que définie ci-dessus, caractérisé en ce que l'on fait agir un composé de formule (II)

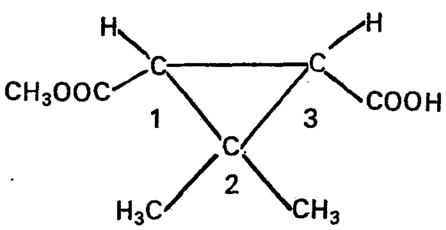

(II)

de configuration (1R,3S) cis ou (1S,3R) cis, avec un agent d'estérification susceptible de transformer le composé (II) en son ester terbutylique, pour obtenir respectivement le composé de formule ($III_A$)

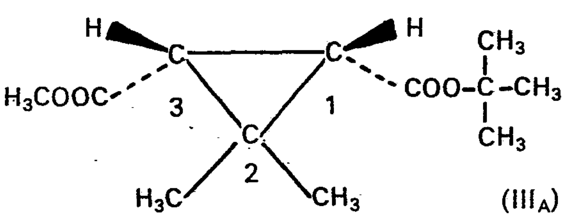

($III_A$)

de configuration (1S,3R), ou le composé de formule ($III_B$)

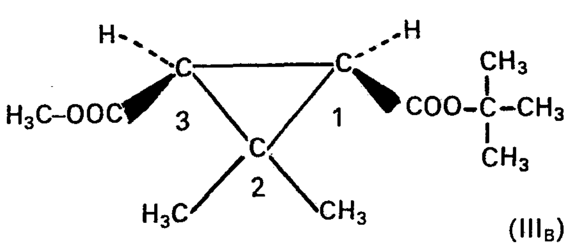

($III_B$)

de configuration (1R,3S), dont on hydrolyse sélectivement la fonction ester méthylique, pour obtenir un composé de formule ($I_A$)

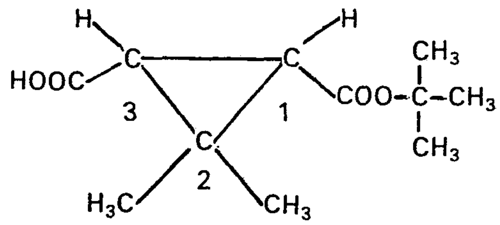

($I_A$)

respectivement de configuration (1S,3R) cis ou (1R,3S) cis, c'est-à-dire inverse de celle du composé de formule (II) de départ, composé de formule ($I_A$) que, le cas échéant, l'on isomérise par action d'une base forte, pour obtenir un composé de formule ($I_B$)

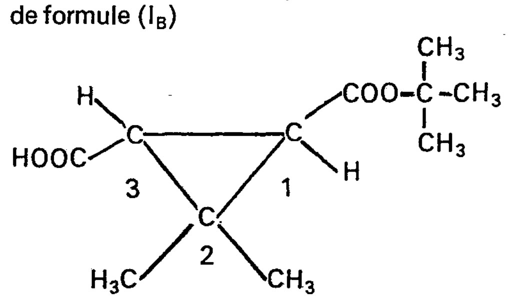

($I_B$)

respectivement de configuration (1R,3R) trans ou (1S,3S) trans.

L'invention a donc notamment pour objet, un procédé de préparation des composés de formule (I), tels que définis ci-dessus, de configuration cis, caractérisé en ce que l'on fait agir un composé de formule (II)

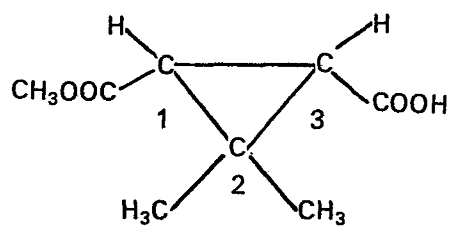

(II)

de configuration (1R,3S) ci ou (1S,3R) cis, avec un agent d'estérification susceptible de transformer le composé (II) en son ester terbutylique, pour obtenir respectivement le composé de formule ($III_A$)

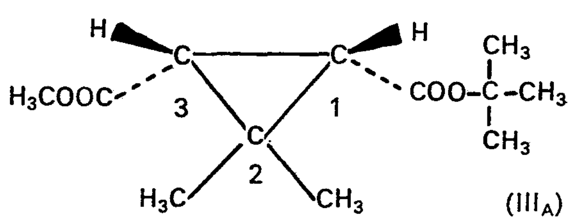

($III_A$)

de configuration (1S,3R), ou le composé de formule ($III_B$)

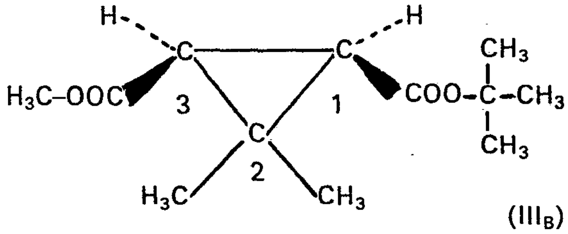

$(III_B)$

de configuration (1R,3S), dont on hydrolyse sélectivement la fonction ester méthylique, pour obtenir un composé de formule $(I_A)$

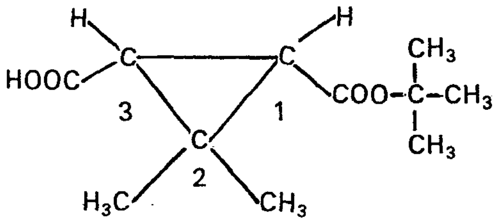

$(I_A)$

respectivement de configuration (1S,3R) cis ou (1R,3S) cis, c'est-à-dire inverse de celle du composé de formule (II) de départ, ainsi qu'un procédé de préparation des composés de formule (I), tels que définis ci-dessus, de configuration trans, caractérisé en ce que l'on fait agir un composé de formule (II)

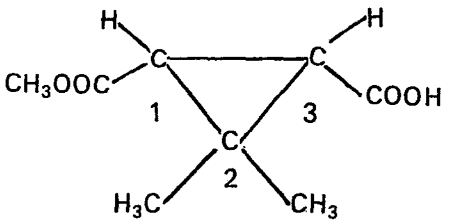

(II)

de configuration (1R,3S) cis ou (1S,3R) cis, avec un agent d'estérification susceptible de transformer le composé (II) en son ester terbutylique, pour obtenir respectivement le composé de formule $(III_A)$

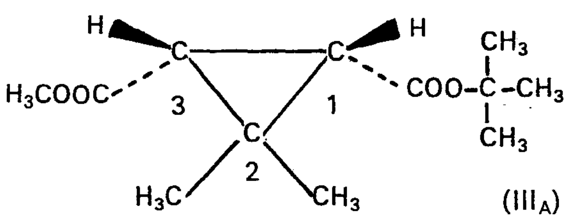

$(III_A)$

de configuration (1S,3R), ou le composé de formule $(III_B)$

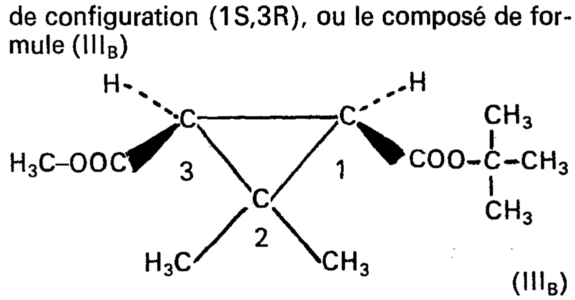

$(III_B)$

de configuration (1R,3S), dont on hydrolyse sélectivement la fonction ester méthylique, pour obtenir un composé de formule $(I_A)$

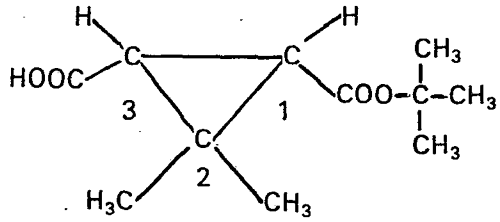

$(I_A)$

respectivement de configuration (1S,3R) cis ou (1R,3S) cis, c'est-à-dire inverse de celle du composé de formule (II) de départ, composé de formule $(I_A)$ que l'on isomérise par action d'une base forte pour obtenir un composé de formule $(I_B)$

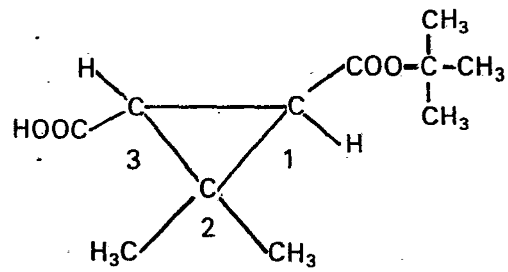

$(I_B)$

respectivement de configuration (1R,3R) trans ou (1S,3S) trans.

Dans un mode d'exécution préféré du procédé de l'invention,

– l'agent d'estérification par l'intermédiaire duquel on transforme le composé (II) en son ester terbutylique est l'isobutène utilisé en présence d'un acide, de préférence l'acide sulfurique, au sein d'un solvant organique, de préférence le chlorure de méthylène;

– l'hydrolyse sélective de la fonction ester méthylique du composé $(III_A)$ ou $(III_B)$ est effectuée par une base minérale, de préférence le carbonate de potassium, au sein d'un solvant hydroalcoolique, de préférence hydrométhanolique;

– la base forte utilisée dans l'isomérisation est un alcoolate alcalin, tel que le méthylate de sodium, l'éthylate de sodium ou le terbutylate de potassium et l'on opère au sein d'un alcool ou d'un éther.

A la connaissance de la demanderesse, aucun procédé n'a été décrit à ce jour, pour la transformation stéréosélective de dérivés cyclopropane dicarboxyliques.

La transformation stéréosélective selon l'invention des composés de formule (II) de configuration (1R,3S) cis et (1S,3R) cis en composés de formule $(I_A)$ respectivement de configuration (1S,3R) cis et (1R,3S) cis a été réalisée par des moyens originaux par rapport à ceux habituellement utilisés pour certains dérivés d'acides cyclopropane carboxyliques.

Le procédé permettant d'obtenir cette transformation stéréosélective est simple et met à profit la structure dicarboxylique des dérivés, et par là le fait que la simple interconversion des groupements acide et ester conduit à la transformation stéréosélective souhaitée.

Le schéma figurant ci-joint explicite l'ensemble des transformations réalisées.

L'invention a enfin pour objet à titre de produits intermédiaires nécessaires à la préparation des composés de formule (I), les composés de formule ($III_A$) et ($III_B$) définis ci-dessus.

Les composés de formule (II) de configuration (1R,3S) cis et (1S,3R) cis sont des composés nouveaux. Leur préparation figure ci-après dans la partie expérimentale et est décrite dans une demande de brevet déposé ce jour par la Société demanderesse et intitulée «Nouveaux esters méthyliques dérivés de l'acide 2,2-diméthyl cyclopropane 1,3-dicarboxylique, leur préparation et les nouveaux intermédiaires obtenus».

Les composés de formule (I) sont des composés intermédiaires, par exemple dans la synthèse des dérivés chrysanthémiques correspondants. Il est en effet possible, à partir des composés (I), par une suite de réactions comprenant une réduction de la fonction acide, puis une oxydation de l'alcool obtenu en aldéhyde et enfin une réaction de Wittig à l'aide d'un halogènure de triphénylisopropyl phosphonium, d'obtenir l'ester chrysanthémique terbutylique dont la configuration est celle du composé (I) utilisé.

Une suite de réactions de ce type, s'appliquant à des esters méthyliques racémiques, a déjà été décrite par la société demanderesse dans le brevet belge n° 862 461.

Les esters chrysanthémiques terbutyliques ainsi obtenu sont eux-mêmes des intermédiaires dans la synthèse d'autres esters connus possédant des propriétés pesticides, notamment insecticides.

Les exemples suivants illustrent l'invention sans toutefois la limiter:

Exemple 1

Mono ester terbutylique de l'acide (1R,3S) cis 2,2-diméthyl cyclopropane 1,3-dicarboxylique.

Stade A: Ester terbutylique de l'acide (1R,3S) cis 2,2-diméthyl 1-méthoxy carbonyl cyclopropane-3-carboxylique.

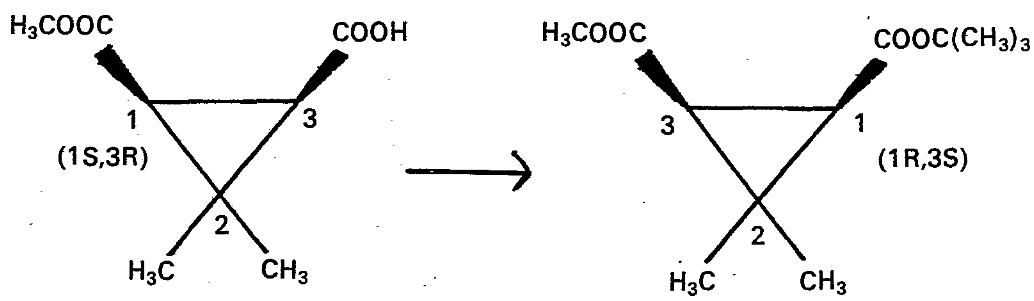

On dissout 0,516 g de mono ester méthylique de l'acide (1S,3R) cis 2,2-diméthyl cyclopropane 1,3-dicarboxylique dans 10 $cm^3$ de chlorure de méthylène, ajoute quelques gouttes d'acide sulfurique et fait passer un courant d'isobutène dans la solution pendant 45 minutes. On ajoute ensuite une solution saturée de bicarbonate de sodium, extrait à l'éther, lave la phase éthérée à l'eau puis avec une solution aqueuse de chlorure de sodium, sèche et évapore la solvant. On obtient 0,51 g de diester attendu.

$[\alpha]_D^{25} = -22{,}06°$ (Acétone).

Spectre R M N ($CCL_4$)

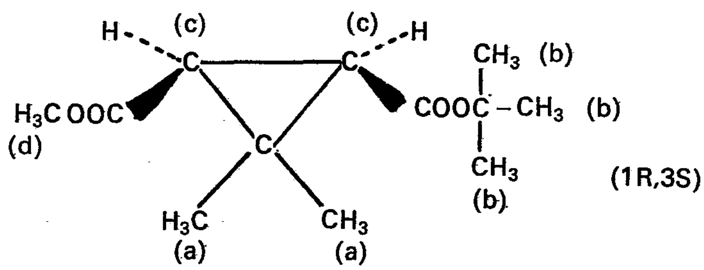

δ Ha: singulet à 1,16 ppm
singulet à 1,29 ppm
δ Hb: singulet à 1,35 ppm
δ Hc: singulet à 1,66 ppm
δ Hd: singulet à 3,56 ppm

Analyse

| | | |
|---|---|---|
| Calculé | C% 63,14 | H% 8,83 |
| Trouvé | 63,5 | 8,8 |

Stade B: Mono ester terbutylique de l'acide (1R,3S) cis 2,2-diméthyl cyclopropane 1,3-dicarboxylique.

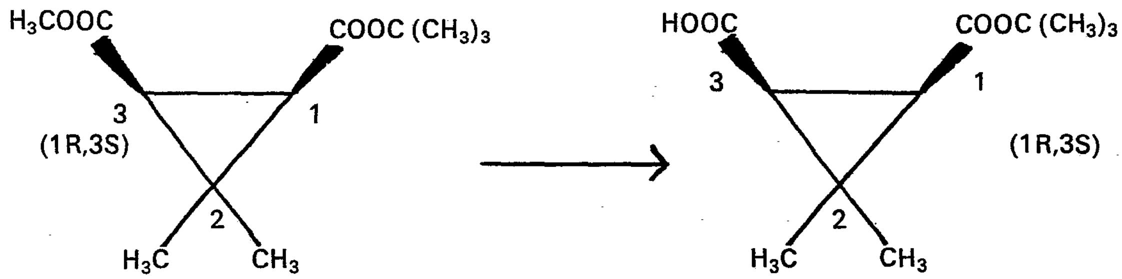

(1R,3S) (1R,3S)

On dissout 0,42 g de diester obtenu au stade A, dans 7 $cm^3$ de mélange méthano-eau (1/1). On ajoute 1,27 g de carbonate de potassium, porte au reflux pendant 16 heures, laisse revenir à température ambiante puis ajoute de l'acide chlorhydrique à 10% (pH 2), extrait à l'éther, lave la phase éthérée à l'eau, puis avec une solution aqueuse saturée de chlorure de sodium, sèche et évapore le solvant. On purifie le produit sur silice (éluant: éther. Rf=0,85) et obtient 0,31 g de produit attendu.

$[\alpha]_D^{20}$ = +1,78° (Ethanol) F=114 °C.

Spectre IR
- absorption de 2400 à 3600 $cm^{-1}$ (OH),
- absorption à 1720 $cm^{-1}$ (C=O ester),
- absorption à 1705 $cm^{-1}$ (c=O acide).

Spectre R M N ($CDCl_3$)

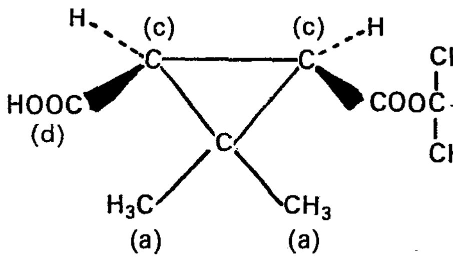

(1R,3S)

δ Ha: singulet à 1,24 ppm
singulet à 1,36 ppm
δ Hb: singulet à 1,42 ppm
δ Hc: singulet à 1,87 ppm
δ Hd: massif centré sur 10,7 ppm

Le mono ester méthylique de l'acide (1S,3R) cis 2,2-diméthyl cyclopropane 1,3-dicarboxylique ainsi que son analogue (1R,3S) cis peuvent être obtenus de la manière suivante:

a) – isolement du mono ester de l'acide (1R,3S) cis.

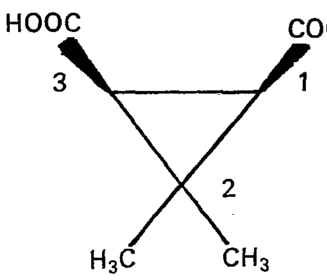

(1R,3S)

On met en solution dans 100 $cm^3$ d'acétone, 5,26 g de mono ester méthylique de l'acide (1RS,3SR) cis 2,2-diméthyl cyclopropane 1,3-dicarboxylique, ajoute 3,7 g de d (+) α-méthyl benzylamine puis laisse au repos à température ambiante pendant 4 jours et filtre les cristaux formés. On évapore à sec la filtrat et le reprend à l'acétone. On laisse au repos pendant 24 heures et filtre les cristaux formés que l'on joint à ceux précédemment obtenus. Le filtrat (A) est conservé.

On dissout ces cristaux dans l'acétone, puis après avoir initié la cristallisation, laisse au repos pendant 3 jours. On filtre les cristaux formés, les sèche et obtient 1,69 g de sel attendu.

On dissout le sel dans l'eau, ajoute 1,03 g de carbonate de potassium, lave la phase aqueuse à l'éther, l'acidifie, légèrement par addition d'acide chlorhydrique à 10%, extrait à l'éther, lave la phase éthérée à l'eau puis à l'eau salée, la sèche et évapore le solvant. On obtient 1 g de mono ester méthylique de l'acide (1R,3S) cis 2,2-diméthyl cyclopropane 1,3-dicarboxylique.

$[\alpha]_D^{25}$ = +30,92° (Ethanol) F=54 °C, qui se révèle être un mélange renfermant 94,23% de mono ester de l'acide (1R,3S) cis et 5,77% de mono ester de l'acide (1S,3R) cis.

b) – isolement du mono ester de l'acide (1S,3R) cis.

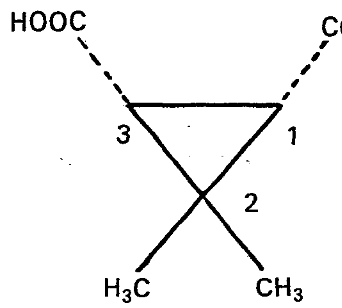

(1S,3R)

On évapore à sec la filtrat (A) obtenu précédemment, puis le traite comme décrit ci-dessus pour obtenir l'acide correspondant, qui se révèle être un mélange renfermant 57% de mono ester de l'acide (1S,3R) cis et 43% de mono ester de l'acide (1R,3S) cis.

On traite cet acide (2,56 g) par 1,8 g de 1(–) α-méthyl benzylamine puis poursuit les opérations comme décrit précédemment dans la préparation de l'acide (1R,3S) cis. On obtient finalement 0,813 g d'acide (1S,3R) cis attendu,

$[\alpha]_D^{25}$ = –29,35° (Ethanol), F=53 °C qui se révèle être un mélange renfermant 92% de mono

ester de l'acide (1S,3R) cis et 8% de mono ester de l'acide (1R,3S) cis.

Spectre RMN ($CDCl_3$)

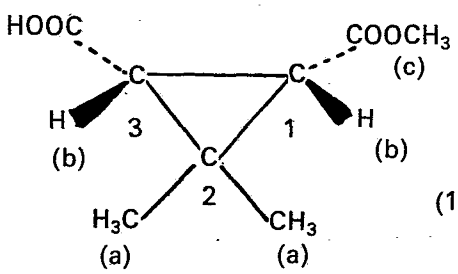

(1S,3R)

δ Ha = singulet à 1,28 ppm
singulet à 1,40 ppm
δ Hb = singulet à 1,96 ppm
δ = singulet à 3,70 ppm

Exemple 2

Mono ester terbutylique de l'acide (1S,3S) trans 2,2-diméthyl cyclopropane 1,3-dicarboxylique.

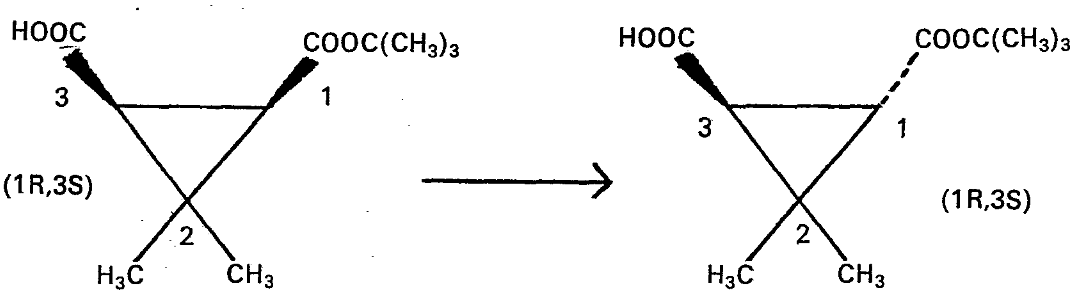

(1R,3S) (1R,3S)

On mélange sous gaz inerte 0,64 g de mono ester terbutylique de l'acide (1R,3S) cis 2,2-diméthyl cyclopropane 1,3-dicarboxylique et 3 $cm^3$ d'une solution 5 M de méthylate de sodium dans le méthanol. On porte au reflux pendant 1 heure 15 minutes, laisse revenir à température ambiante puis additionne d'acide chlorhydrique à 10% (pH 2). On extrait à l'éther, lave la phase éthérée à l'eau, puis avec une solution aqueuse saturée de chlorure de sodium, sèche, évapore le solvant et obtient 0,35 g de mono ester terbutylique de l'acide (1S,3S) trans attendu.
F = 105 °C

Spectre IR
- absorption de 2400 à 3680 $cm^{-1}$ (OH),
- absorption à 1720 $cm^{-1}$ (C=O ester),
- absorption à 1700 $cm^{-1}$ (C=O acide).

Spectre RMN ($CDCl_3$)

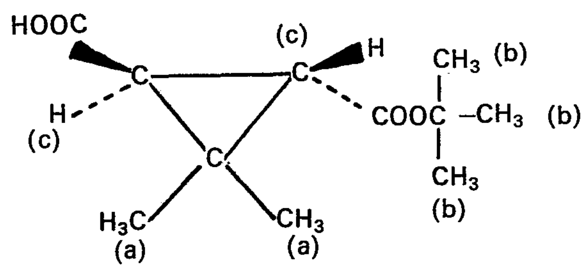

δ Ha: singulet à 1,30 ppm
singulet à 1,32 ppm
δ Hb: singulet à 1,43 ppm
δ Hc: singulet à 2,20 ppm

Exemple 3

Mono ester terbutylique de l'acide (1S,3R) cis 2,2-diméthyl cyclopropane 1,3-dicarboxylique.

Stade A: Ester terbutylique de l'acide (1S,3R) cis 2,2-diméthyl 1-méthoxy carbonyl cyclopropane-3-carboxylique.

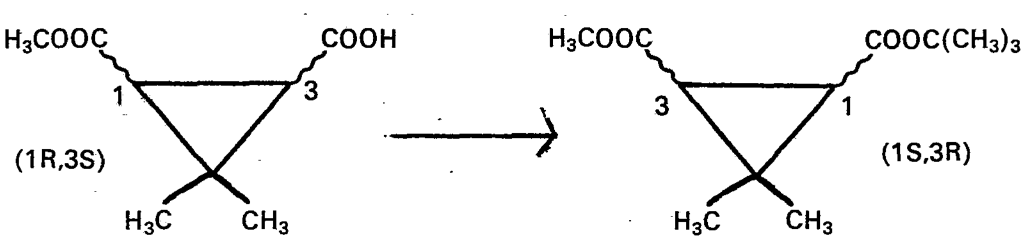

(1R,3S) (1S,3R)

On opère de manière analogue à celle décrite au Stade A de l'exemple 1, au départ du mono ester méthylique de l'acide (1R,3S) cis 2,2-diméthyl cyclopropane 1,3-dicarboxylique et obtient le diester attendu.

Spectre RMN ($CCl_4$)

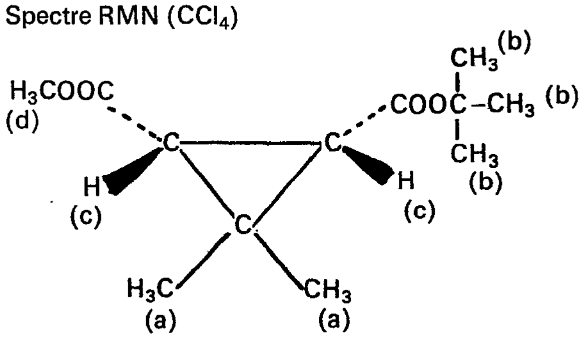

δ Ha: singulet à 1,16 ppm
singulet à 1,29 ppm
δ Hb: singulet à 1,35 ppm
δ Hc: singulet à 1,66 ppm
δ Hd: singulet à 3,56 ppm.

Stade B: Monoester terbutylique de l'acide (1S,3R) cis 2,2-diméthyl cyclopropane-1,3-dicarboxylique

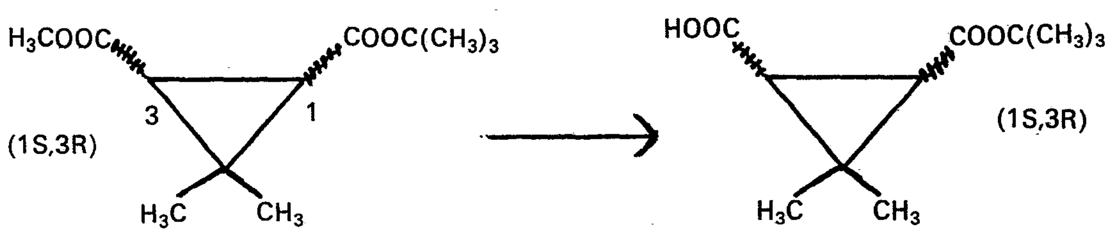

On opère de manière analogue à celle décrite au Stade B de l'exemple 1, au départ du diester obtenu au stade A ci-dessus et obtient le produit attendu.

$[\alpha]_D^{20} = -1{,}8°$ (éthanol)

Exemple 4

Mono ester terbutylique de l'acide (1R,3R) trans 2,2-diméthyl cyclopropane 1,3-dicarboxylique.

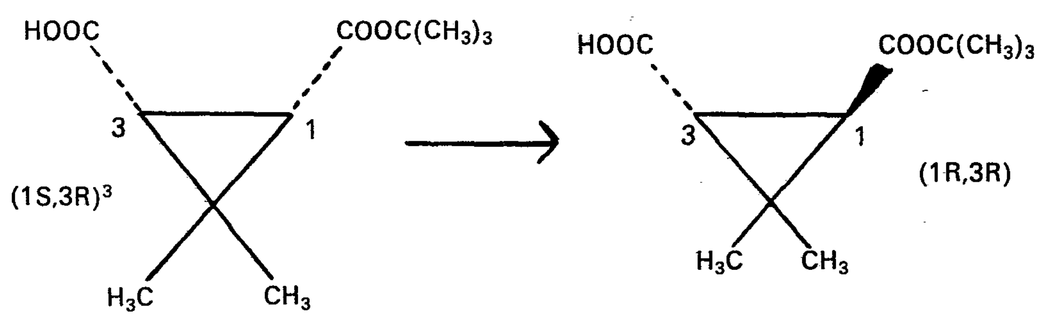

On mélange sous gaz inerte 0,31 g de terbutylate de potassium, 0,21 g de mono ester terbutylique de l'acide (1S,3R) cis 2,2-diméthyl cyclopropane 1,3-dicarboxylique et 45 $cm^3$ de tétrahydrofuranne. On porte au reflux pendant 1 heure puis laisse revenir à température ambiante et ajoute une solution d'acide chlorhydrique à 10%. On extrait à l'éther, lave la phase éthérée à l'eau puis avec une solution aqueuse saturée de chlorure de sodium, sèche, évapore le solvant et obtient 0,13 g de mono ester terbutylique de l'acide (1R,3R) trans attendu.

$[\alpha]_D^{20} = -29{,}90°$ (éthanol).

**Revendications pour les Etats contractants BE, CH, DE, GB, IT, LI, LU, NL, SE**

1. Les composés répondant à la formule (I)

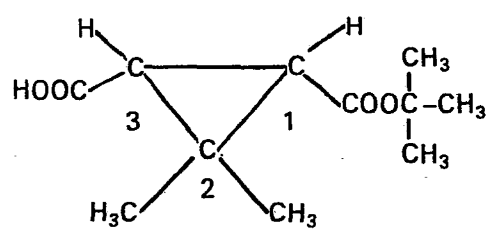

(I)

de configuration cis ou trans, sous leurs formes optiquement actives.

2. Les mono esters terbutyliques des acides (1R,3S) et (1S,3R) cis 2,2-diméthyl cyclopropane 1,3-dicarboxyliques.

3. Les mono esters terbutyliques des acides (1R,3R) et (1S,3S) trans 2,2-diméthyl cyclopropane 1,3-dicarboxyliques.

4. Procédé de préparation des composés de formule (I), tels que définis à la revendication 1, caractérisé en ce que l'on fait agir un composé de formule (II)

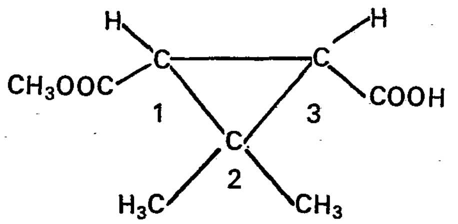

(II)

de configuration (1R,3S) cis ou (1S,3R) cis, avec un agent d'estérification susceptible de transformer le composé (II) en son ester terbutylique, pour obtenir respectivement le composé de formule ($III_A$)

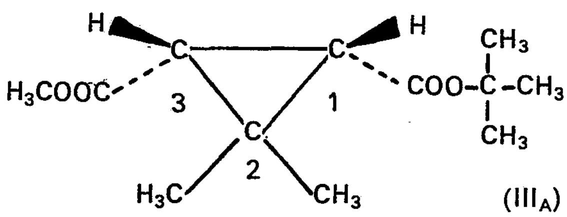

($III_A$)

de configuration (1S,3R), ou le composé de formule ($III_B$):

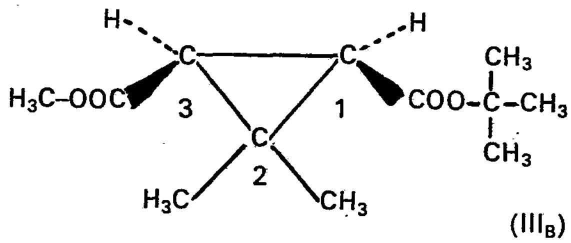

($III_B$)

de configuration (1R,3S), dont on hydrolyse sélectivement la fonction ester méthylique, pour obtenir un composé de formule ($I_A$)

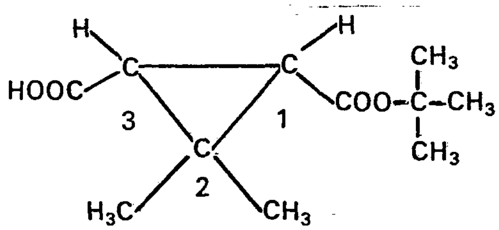

($I_A$)

respectivement de configuration (1S,3R) cis ou (1R,3S) cis, c'est-à-dire inverse de celle du composé de formule (II) de départ, composé de formule ($I_A$) que, le cas échéant l'on isomèrise par action d'une base forte, pour obtenir un composé de formule ($I_B$)

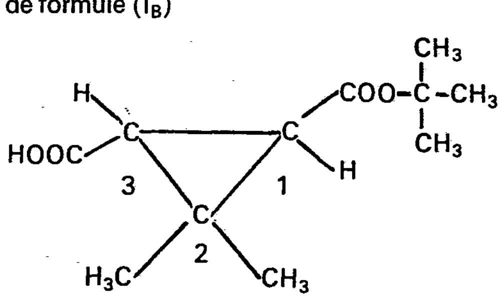

($I_B$)

respectivement de configuration (1R,3R) trans ou (1S,3S) trans.

5. Procédé selon la revendication 4, caractérisé en ce que,

– l'agent d'estérification par l'intermédiaire duquel on transforme le composé (II) en son ester terbutylique est l'isobutène utilisé en présence d'un acide, de préférence l'acide sulfurique, au sein d'un solvant organique, de préférence le chlorure de méthylène;

– l'hydrolyse sélective de la fonction ester méthylique du composé ($III_A$) ou ($III_B$) est effectuée par une base minérale, de préférence le carbonate de potassium, au sein d'un solvant hydroalcoolique, de préférence hydrométhanolique;

– la base forte utilisée dans l'isomérisation est un alcoolate alcalin, tel que le méthylate de sodium, l'éthylate de sodium, ou le terbutylate de potassium et l'on opère au sein d'un alcool ou d'un éther.

6. A titre de produits intermédiaires, nécessaires à la préparation des composés de formule (I), les composés de formules ($III_A$) et ($III_B$) définis à la revendication 4.

**Revendications pour l'Etat contractant AT**

1. Procédé de préparation des composés répondant à la formule (I):

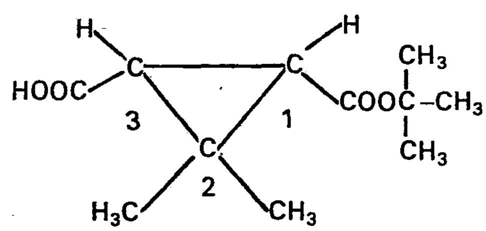

(I)

de configuration cis ou trans, sous leurs formes optiquement actives, caractérisé en ce que l'on fait agir un composé de formule (II):

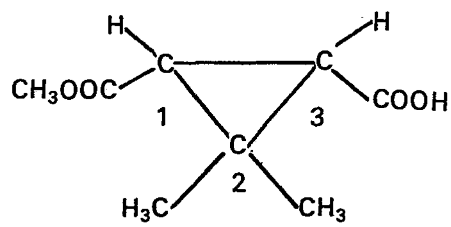

(II)

de configuration (1R,3S) cis ou (1S,3R) cis, avec un agent d'estérification susceptible de transformer le composé (II) en son ester terbutylique, pour obtenir respectivement le composé de formule ($III_A$)

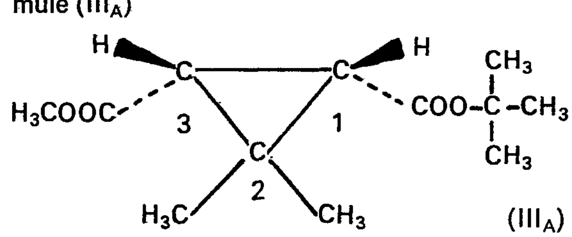

($III_A$)

de configuration (1S,3R), ou le composé de formule ($III_B$):

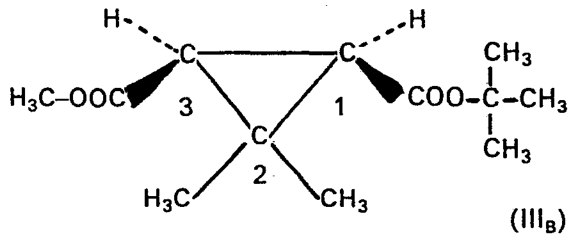

($III_B$)

de configuration (1R,3S), dont on hydrolyse sélectivement la fonction ester méthylique, pour obtenir un composé de formule ($I_A$)

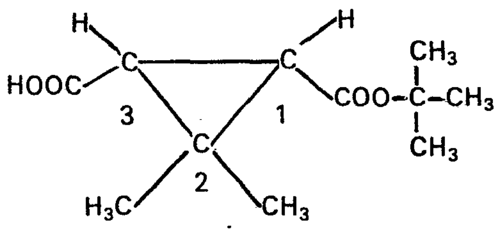

($I_A$)

respectivement de configuration (1S,3R) cis ou (1R,3S) cis, c'est-à-dire inverse de celle du composé de formule (II) de départ, composé de formule ($I_A$) que, le cas échéant, l'on isomérise par action d'une base forte, pour obtenir un composé de formule ($I_B$)

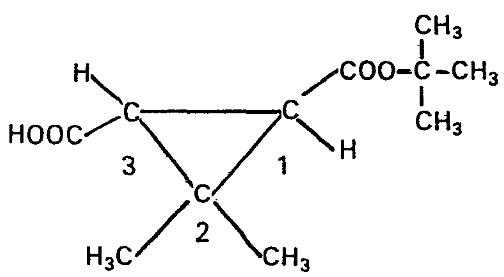

($I_B$)

respectivement de configuration (1R,3R) trans ou (1S,3S) trans.

2. Procédé selon la revendication 1, caractérisé en ce que,

– l'agent d'estérification par l'intermédiaire duquel on transforme le composé (II) en son ester terbutylique est l'isobutène utilisé en présence d'un acide, de préférence l'acide sulfurique, au sein d'un solvant organique, de préférence le chlorure de méthylène;

– l'hydrolyse sélective de la fonction ester méthylique du composé ($III_A$) ou ($III_B$) est effectuée par une base minérale, de préférence le carbonate de potassium, au sein d'un solvant hydroalcoolique, de préférence hydrométhanolique;

– la base forte utilisée dans l'isomérisation est un alcoolate alcalin, tel que le méthylate de sodium, l'éthylate de sodium, ou le terbutylate de potassium et l'on opère au sein d'un alcool.

3. Procédé selon la revendication 2, caractérisé en ce que la base forte utilisée dans l'isomérisation est un alcoolate alcalin, tel que le méthylate de sodium, l'éthylate de sodium ou le terbutylate de potassium et l'on opère au sein d'un alcool ou d'un éther.

4. Procédé selon la revendication 1, pour préparer les mono esters terbutyliques des acides (1R,3S) et (1S,3R) cis 2,2-diméthyl cyclopropane 1,3-dicarboxyliques, caractérisé en ce que l'on fait agir un composé de formule (II):

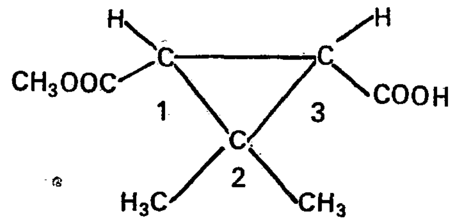

(II)

de configuration (1R,3S) cis ou (1S,3R) cis, avec un agent d'estérification susceptible de transformer le composé (II) en son ester terbutylique, pour obtenir respectivement le composé de formule ($III_A$)

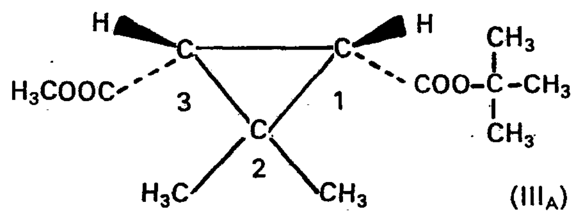

($III_A$)

de configuration (1S,3R), ou le composé de formule ($III_B$):

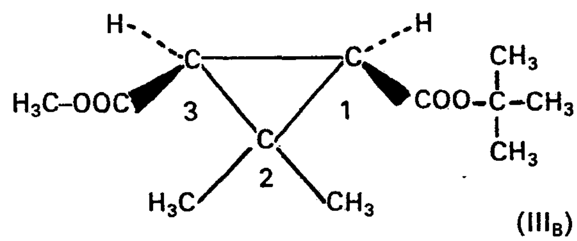

($III_B$)

de configuration (1R,3S), dont on hydrolyse sélectivement la fonction ester méthylique, pour obtenir un composé de formule ($I_A$)

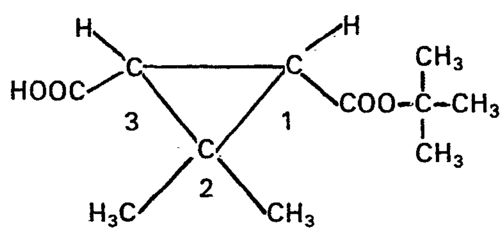

($I_A$)

respectivement de configuration (1S,3R) cis ou (1R,3S) cis, c'est-à-dire inverse de celle du composé de formule (II) de départ.

5. Procédé selon la revendication 1, pour préparer les mono esters terbutyliques des acides (1R,3R) et (1S,3S) trans 2,2-diméthyl cyclopropane 1,3-dicarboxyliques, caractérisé en ce que l'on fait agir un composé de formule (II):

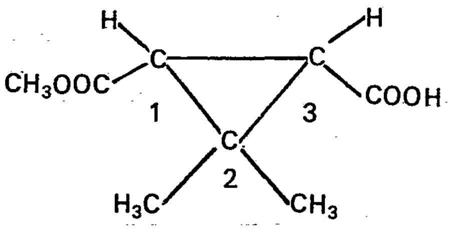

(II)

de configuration (1R,3S) cis ou (1S,3R) cis, avec un agent d'estérification susceptible de transformer le composé (II) en son ester terbutylique, pour obtenir respectivement le composé de formule ($III_A$)

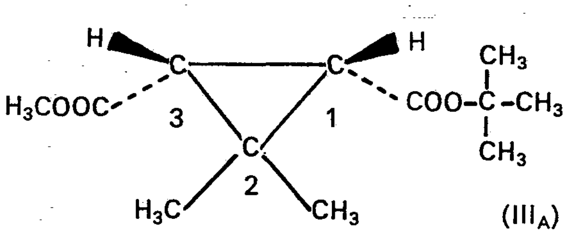

($III_A$)

de configuration (1S,3R), ou le composé de formule ($III_B$):

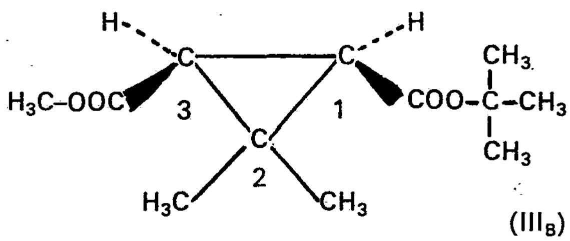

($III_B$)

de configuration (1R,3S) dont on hydrolyse sélectivement la fonction ester méthylique, pour obtenir un composé de formule ($I_A$)

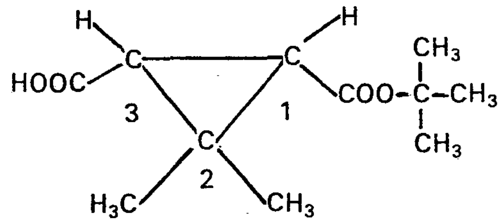

($I_A$)

respectivement de configuration (1S,3R) cis ou (1R,3S) cis, c'est-à-dire inverse de celle du composé de formule (II) de départ, que l'on isomérise par action d'une base forte, pour obtenir un composé de formule ($I_B$):

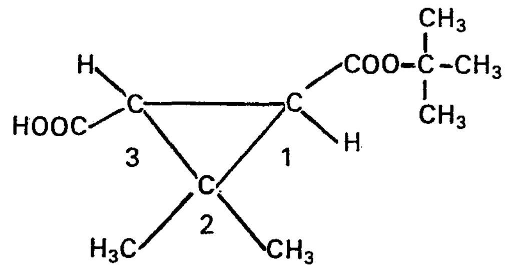

($I_B$)

respectivement de configuration (1R,3R) trans ou (1S,3S) trans.

Planche unique

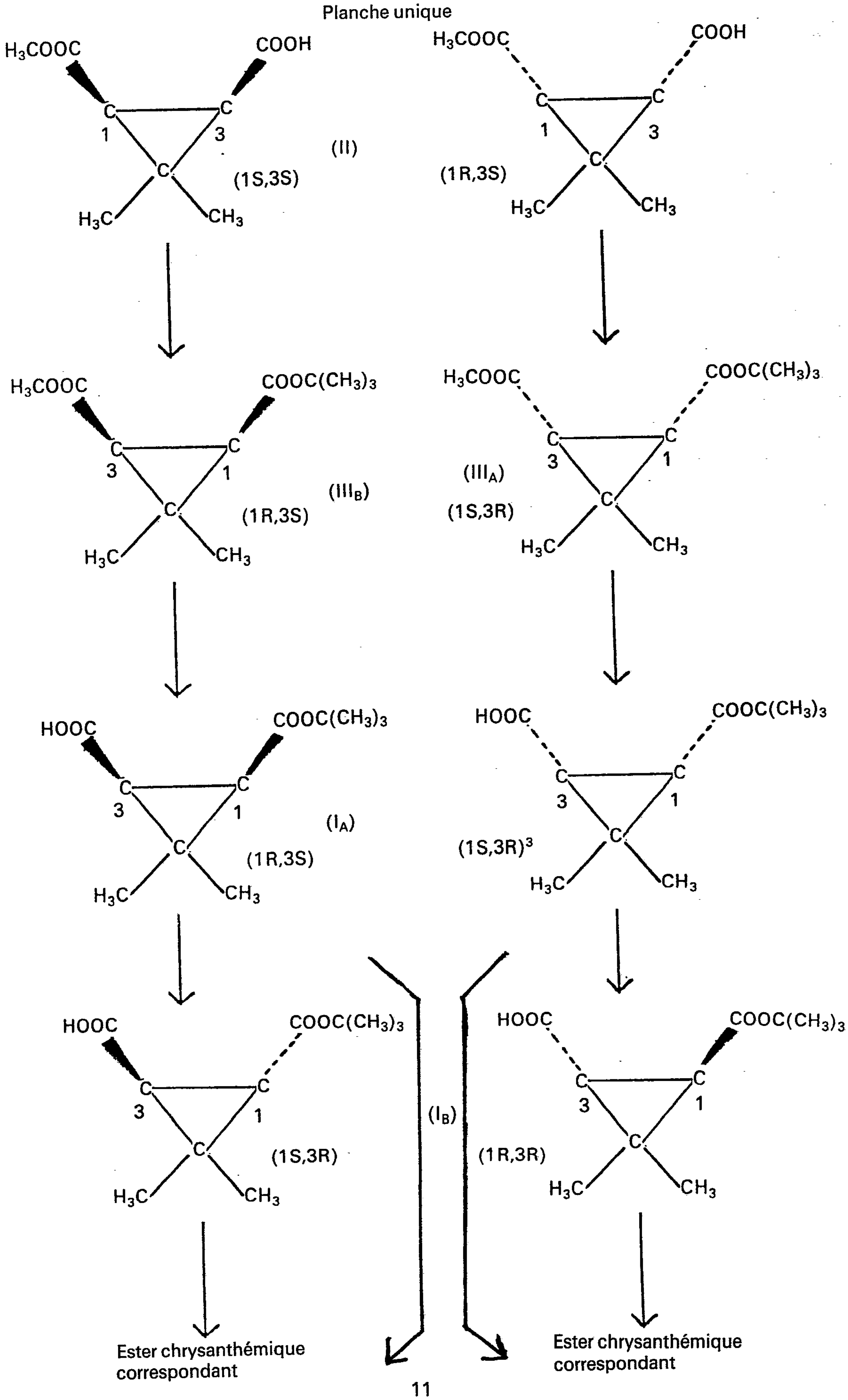

(II)

(1S,3S)

(1R,3S)

($III_B$)

(1R,3S)

($III_A$)

(1S,3R)

($I_A$)

(1R,3S)

(1S,3R)[3]

(1S,3R)

($I_B$)

(1R,3R)

Ester chrysanthémique correspondant

Ester chrysanthémique correspondant

**Patentansprüche für die Vertragsstaaten BE, CH, DE, GB, IT, LI, LU, NL, SE**

1. Verbindungen der Formel (I)

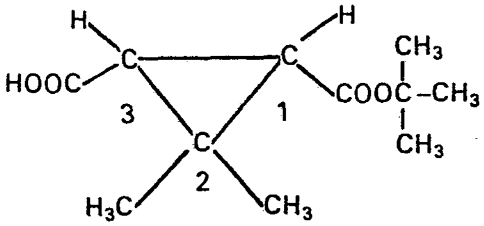
(I)

mit cis- oder trans-Konfiguration in ihren optisch aktiven Formen.

2. Tert.-Butylmonoester der (1R,3S)- und (1S,3R)-cis- 2,2-Dimethyl-cyclopropan-1,3- dicarbonsäuren.

3. Tert.-Butylmonoester der (1R,3R)- und (1S,3S)-trans-2,2-Dimethyl-cyclopropan-1,3-dicarbonsäuren.

4. Verfahren zur Herstellung der Verbindungen der Formel (I) gemäss Anspruch 1, dadurch gekennzeichnet, dass man eine Verbindung der Formel (II)

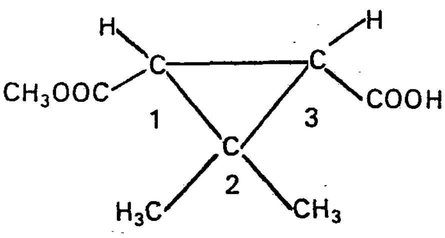
(II)

mit (1R,3S)-cis oder (1S,3R)-cis-Konfiguration mit einem Veresterungsmittel umsetzt, das befähigt ist, die Verbindung (II) in ihren tert.-Butylester zu überführen, um die Verbindung der Formel ($III_A$)

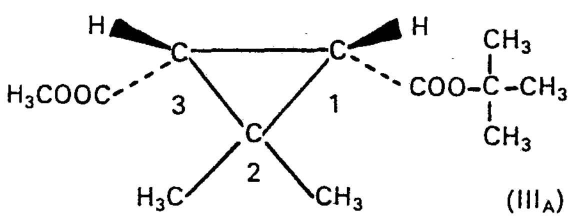
($III_A$)

mit (1S,3R)-Konfiguration bzw. die Verbindung der Formel ($III_B$)

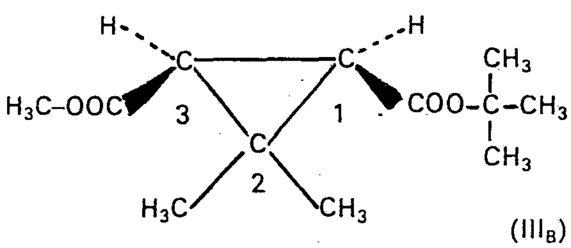
($III_B$)

mit (1R,3S)-Konfiguration zu erhalten, deren Methylesterfunktion man selektiv hydrolysiert, um eine Verbindung der Formel ($I_A$)

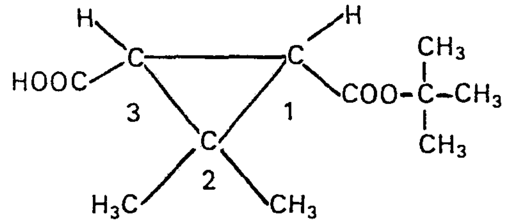
($I_A$)

mit (1S,3R)-cis bzw. (1R,3S)-cis-Konfiguration, d.h. der zu derjenigen der Ausgangsverbindung der Formel (II) inversen Konfiguration zu erhalten, die Verbindung der Formel ($I_A$) gegebenenfalls durch Einwirken einer starken Base isomerisiert, um eine Verbindung der Formel ($I_B$)

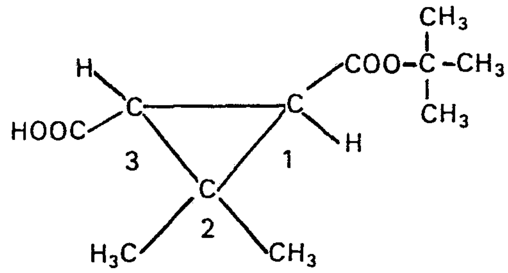
($I_B$)

mit (1R,3R)-trans- bzw. (1S,3S)-trans-Konfiguration zu erhalten.

5. Verfahren gemäss Anspruch 4, dadurch gekennzeichnet, dass das Veresterungsmittel, mit Hilfe dessen man die Verbindung (II) in ihren tert.-Butylester überführt, Isobuten ist, das in Gegenwart einer Säure, vorzugsweise Schwefelsäure, in dem Medium eines organien Lösungsmittels, vorzugsweise Methylenchlorid, verwendet wird;

die selektive Hydrolyse der Methylesterfunktion der Verbindung ($III_A$) oder ($III_B$) mit einer mineralischen Base, vorzugsweise Kaliumcarbonat in dem Medium eines wässrig alkoholischen Lösungsmittels, vorzugsweise wässrigem Methanol, durchgeführt wird;

die bei der Isomerisierung verwendete starke Base, ein Alkalialkoholat wie Natriummethylat, Natriumäthylat, Kalium-tert.-butylat ist und man in dem Medium eines Alkohols oder eines Äthers arbeitet.

6. Als Zwischenprodukte, die für die Herstellung der Verbindungen der Formel (I) erforderlich sind, die Verbindungen der Formeln ($III_A$) und ($III_B$) gemäss Anspruch 4.

**Patentansprüche für den Vertragsstaat AT**

1. Verfahren zur Herstellung der Verbindungen der Formel (I)

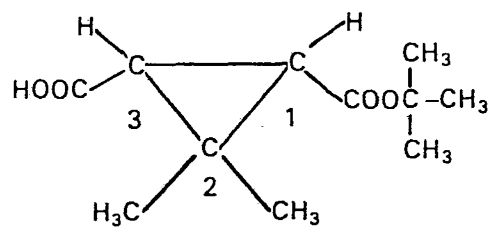
(I)

mit cis- oder trans-Konfiguration in ihren optische aktiven Formen, dadurch gekennzeichnet, dass man eine Verbindung der Formel (II)

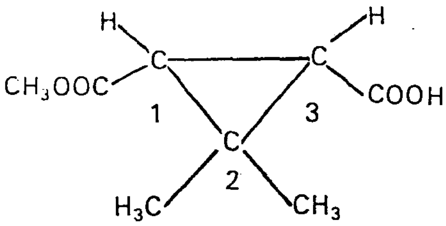

(II)

mit (1R,3S)-cis- oder (1S,3R)-cis-Konfiguration mit einem Veresterungsmittel umsetzt, das befähigt ist, die Verbindung (II) in ihren tert.-Butylester zu überführen, um die Verbindung der Formel ($III_A$)

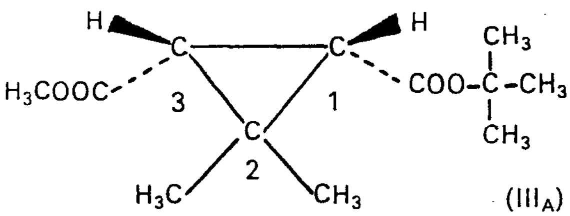

($III_A$)

mit (1S,3R)-Konfiguration bzw. die Verbindung der Formel ($III_B$)

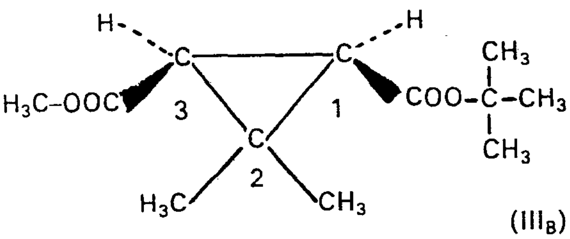

($III_B$)

mit (1R,3S)-Konfiguration zu erhalten, deren Methylesterfunktion man selektiv hydrolysiert, um eine Verbindung der Formel ($I_A$)

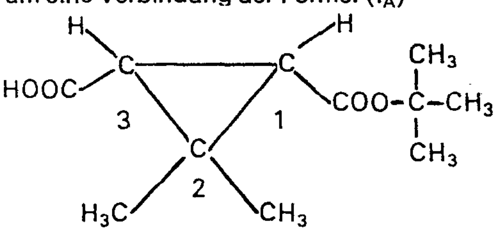

($I_A$)

mit (1S,3R)-cis- bzw. (1R,3S)-cis-Konfiguration, d.h. der zu derjenigen der Ausgangsverbindung der Formel (II) inversen Konfiguration zu erhalten, die Verbindung der Formel ($I_A$) gegebenenfalls durch Einwirken einer starken Base, isomerisiert, um eine Verbindung der Formel ($I_B$)

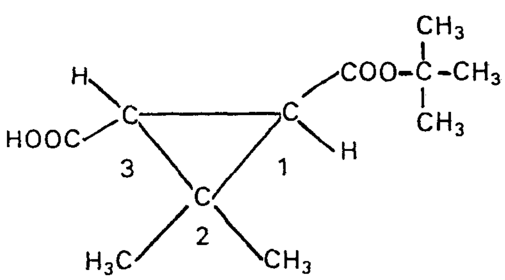

($I_B$)

mit (1R,3R)-trans- bzw. (1S,3S)-trans-Konfiguration zu erhalten.

2. Verfahren gemäss Anspruch 1, dadurch gekennzeichnet, dass das Veresterungsmittel, mit Hilfe dessen man die Verbindung (II) in ihren tert.-Butylester überführt, Isobuten ist, das in Gegenwart einer Säure, vorzugsweise Schwefelsäure, in dem Medium eines organischen Lösungsmittels, vorzugsweise Methylenchlorid, verwendet wird;

die selektive Hydrolyse der Methylesterfunktion der Verbindung ($III_A$) oder ($III_B$) mit einer Mineralbase, vorzugsweise Kaliumcarbonat in dem Medium eines wässrig alkoholischen Lösungsmittels, vorzugsweise wässrigem Methanol, durchgeführt wird;

die bei der Isomerisierung verwendete starke Base, ein Alkalialkoholat, wie Natriummethylat, Natriumäthylat oder Kalium-tert.-butylat ist und man in dem Medium eines Alkohols arbeitet.

3. Verfahren gemäss Anspruch 2, dadurch gekennzeichnet, dass die bei der Isomerisierung verwendete starke Base ein Alkalialkoholat wie Natriummethylat, Natriumäthylat oder Kalium-tert.-butylat ist und man in dem Medium eines Alkohols oder eines Äthers arbeitet.

4. Verfahren gemäss Anspruch 1 zur Herstellung der tert.-Butylester der (1R,3S)- und (1S,3R)-cis-2,2-Dimethyl-cyclopropan-1,3-dicarbonsäuren, dadurch gekennzeichnet,dass man eine Verbindung der Formel (II)

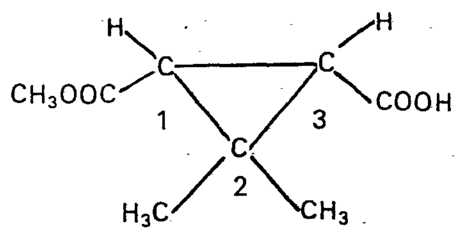

(II)

mit (1R,3S)-cis- oder (1S,3R)-cis-Konfiguration mit einem Veresterungsmittel umsetzt, das befähigt ist, die Verbindung (II) in ihren tert.-Butylester zu überführen, um die Verbindung der Formel ($III_A$)

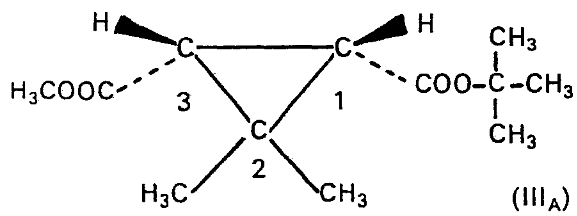

($III_A$)

mit (1S,3R)-Konfiguration bzw. die Verbindung der Formel ($III_B$)

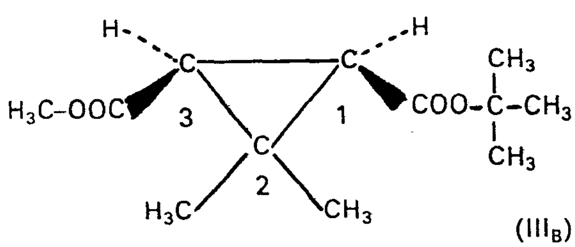

($III_B$)

mit (1R,3S)-Konfiguration zu erhalten, deren Methylesterfunktion man selektiv hydrolysiert, um eine Verbindung der Formel ($I_A$)

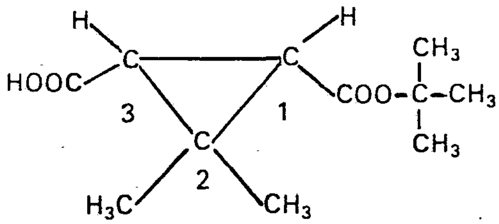

($I_A$)

mit (1S,3R)-cis- bzw. (1R,3S)-cis-Konfiguration, d.h. der zu derjenigen der Ausgangsverbindung der Formel (II) inversen Konfiguration zu erhalten.

5. Verfahren gemäss Anspruch 1 zur Herstellung der Mono-tert.-butylester der (1R,3R)- und (1S,3S)- trans- 2,2- Dimethyl- cyclopropan- 1,3 dicarbonsäuren, dadurch gekennzeichnet, dass man eine Verbindung der Formel (II)

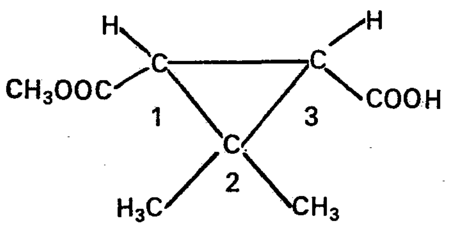

(II)

mit (1R,3S)-cis- oder (1S,3R)-cis-Konfiguration mit einem Veresterungsmittel umsetzt, das befähigt ist, die Verbindung (II) in ihren tert.-Butylester zu überführen, um die Verbindung der Formel ($III_A$)

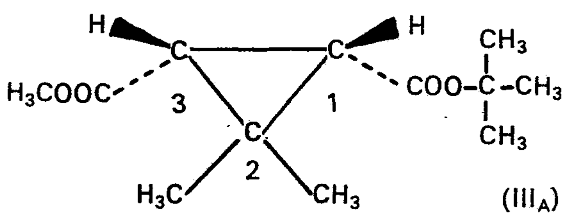

($III_A$)

mit (1S,3R)-Konfiguration bzw. die Verbindung der Formel ($III_B$)

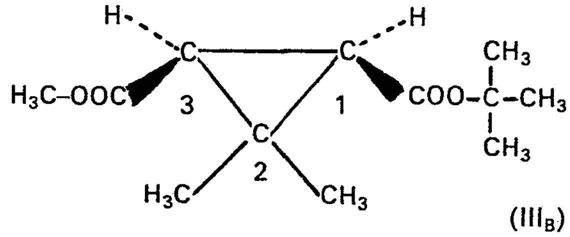

($III_B$)

mit (1R,3S)-Konfiguration zu erhalten, deren Methylesterfunktion man selektiv hydrolysiert, um eine Verbindung der Formel ($I_A$)

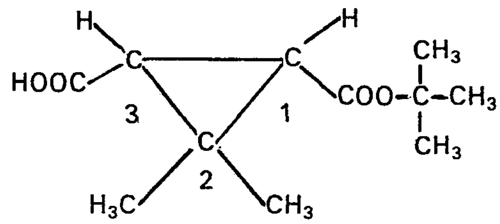

($I_A$)

mit (1S,3R)-cis- bzw. (1R,3S)-cis-Konfiguration, d.h. der zu derjenigen der Ausgangsverbindung der Formel (II) inversen Konfiguration zu erhalten, die man durch Umsetzung mit einer starken Base isomierisiert, um eine Verbindung der Formel ($I_B$)

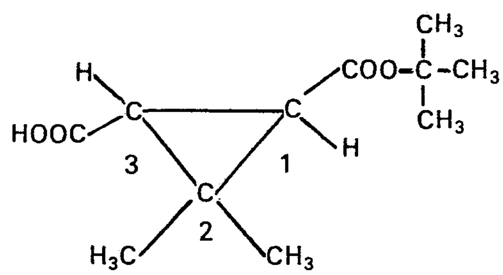

($I_B$)

mit (1R,3R)-trans- bzw. (1S,3S)-trans-Konfiguration zu erhalten.

Einzige Schemaseite

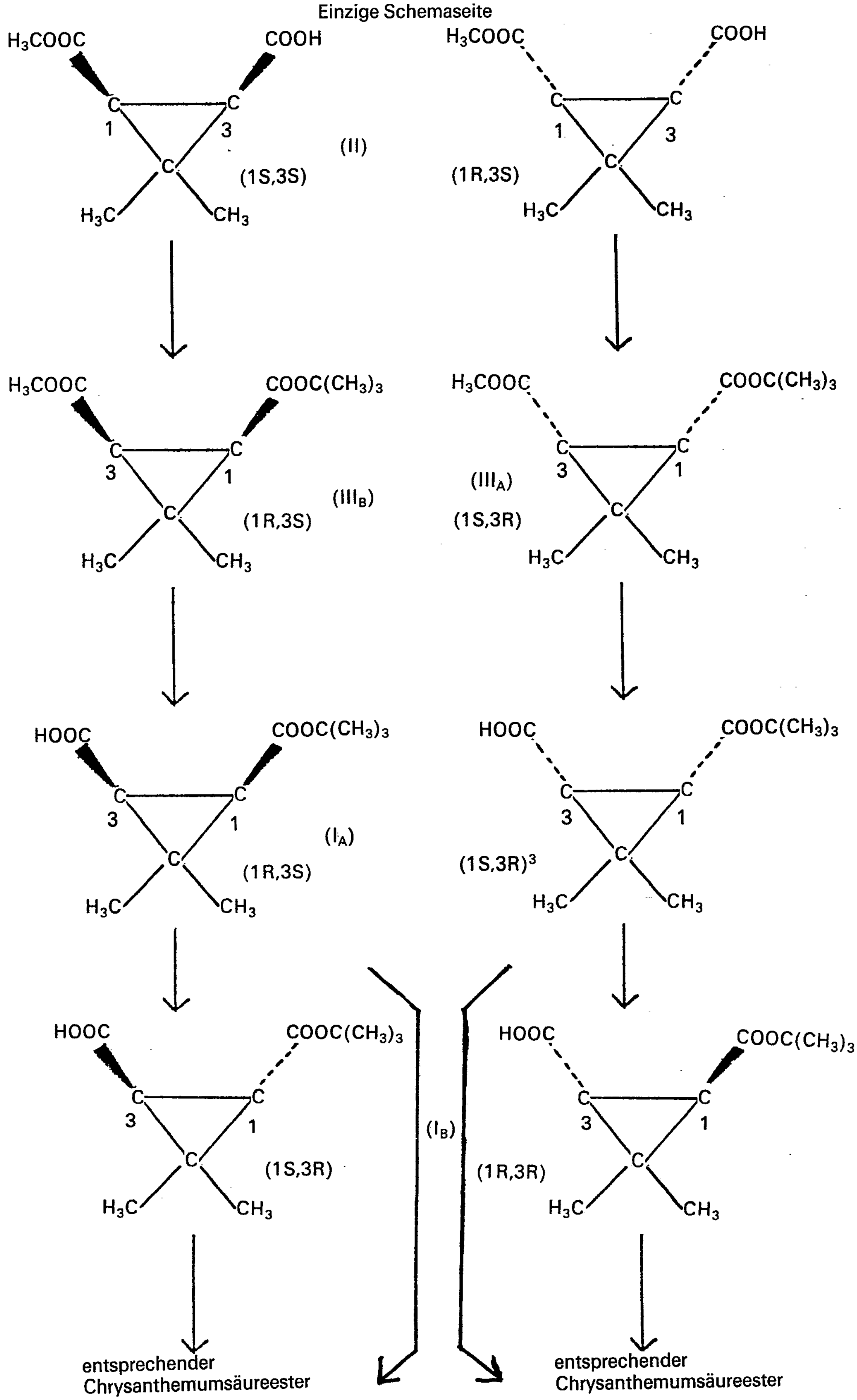


(II) (1S,3S) (1R,3S)

(III_B) (1R,3S) (III_A) (1S,3R)

(I_A) (1R,3S) (1S,3R)³

(I_B) (1S,3R) (1R,3R)

**Claims for the contracting States BE, CH, DE, GB, IT, LI, LU, NL, SE**

1. Compounds responding to the formula (I)

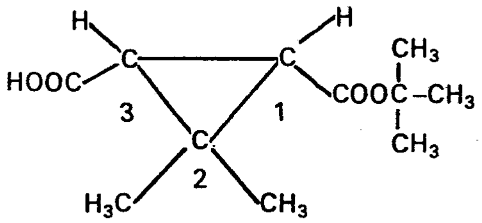

(I)

of cis or trans configuration, in their optically active forms.

2. Mono tert-butyl esters of (1R,3S) and (1S,3R) cis 2,2-dimethylcyclopropane-1,3-dicarboxylic acids.

3. Mono tert-butyl esters of (1R, 3R) and (1S,3S) trans 2,2-dimethylcyclopropane-1,3-dicarboxylic acids.

4. Process for the preparation of the compounds with the formula (I), as defined in Claim 1, characterized in that a compound with the formula (II)

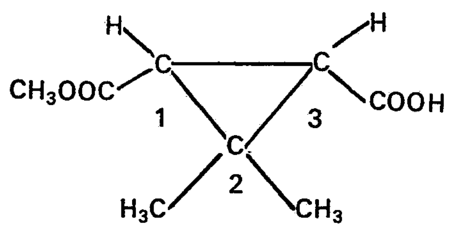

(II)

of (1R,3S) cis or (1S,3R) cis is made to react with an esterification agent able to transform the compound (II) into its tert-butyl ester, in order to obtain respectively the compound with the formula ($III_A$)

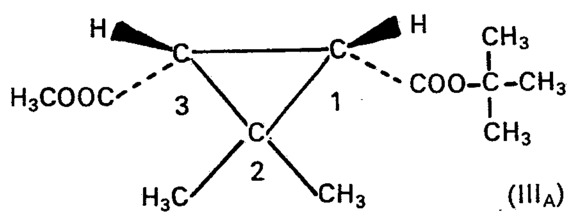

($III_A$)

of (1S,3R) configuration, or the compound with the formula ($III_B$):

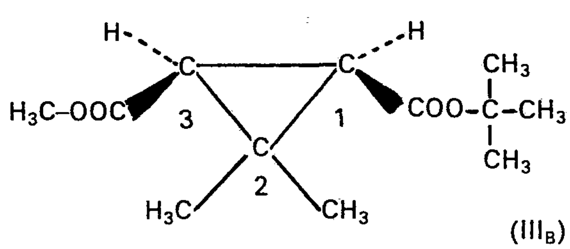

($III_B$)

of (1R,3S) configuration, of which the methyl ester function is selectively hydrolyzed, in order to obtain a compound with the formula ($I_A$)

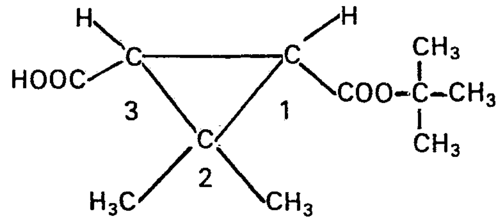

($I_A$)

respectively of configuration (1S,3R) cis or (1R,3S) cis that is to say the inverse of that of the compound with the formula (II) used at the start, which compound with the formula ($I_A$), if necessary, is isomerized by the action of a strong base, in order to obtain a compound with the formula ($I_B$)

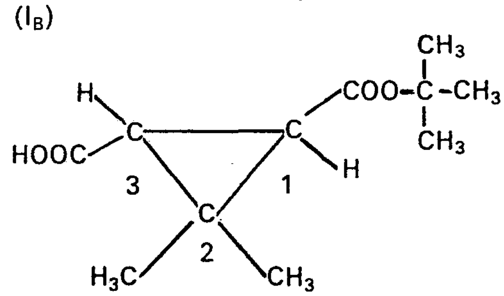

($I_B$)

respectively of (1R,3R) trans or (1S,3S) trans configuration.

5. Process according to Claim 4, characterized in that,

– the esterification agent by the intermediary of which the compound (II) is transformed into its tert-butyl ester is isobutene, utilized in the presence of an acid, preferably sulphuric acid, in an organic solvent, preferably methylene chloride.

– the selective hydrolysis of the methyl ester function of the compound ($III_A$) or ($III_B$) is carried out by a mineral base, preferably potassium carbonate, in a wateralcohol solvent, preferably water-methanol;

– the strong base utilized in the isomerization is an alkaline alcoholate, such as sodium methylate, sodium ethylate, or potassium tert-butylate, and the operation is carried out in an alcohol or an ether.

6. As intermediate products, necessary for the preparation of the compounds with the formula (I), the compounds with the formulae ($III_A$) and ($III_B$) defined in Claim 4.

**Claims for the contracting state AT**

1. Process for the preparation of the compounds responding to the formula (I):

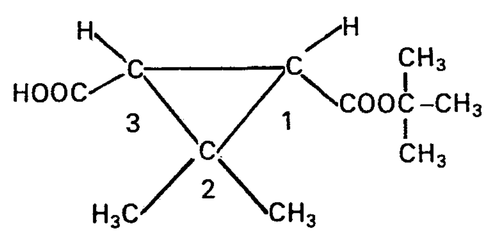

(I)

of cis or trans configuration, in their optically active forms, characterized in that a compound with the formula (II):

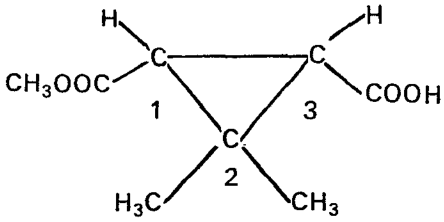

(II)

of (1R,3S) cis or (1S,3R) cis configuration, is made to react with an esterification agent, able to transform the compound (II) into its tert-butyl ester, in order to obtain respectively the compound with the formula ($III_A$):

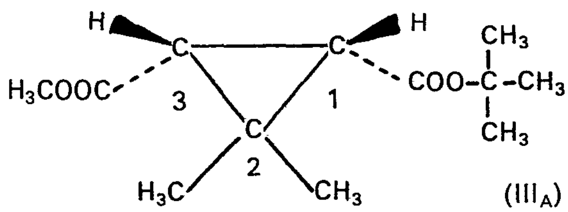

($III_A$)

of (1S,3R) configuration, or the compound with the formula ($III_B$):

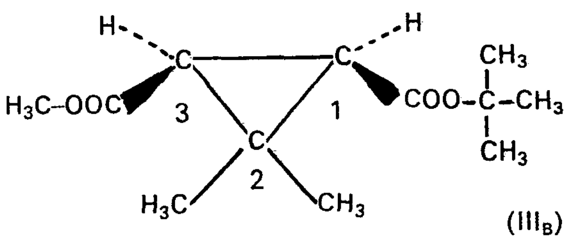

($III_B$)

of (1R,3S) configuration, of which the methyl ester function is selectively hydrolyzed, in order to obtain a compound with the formula ($I_A$):

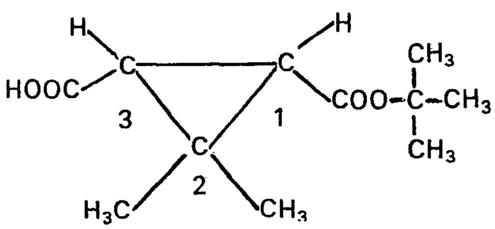

($I_A$)

respectively of (1S,3R) cis or (1R,3S) cis configuration, that is to say, the inverse of that of the compound with the formula (II) used at the start, which compound with the formula ($I_A$), if necessary, is isomerized by the action of a strong base, in order to obtain a compound with the formula ($I_B$)

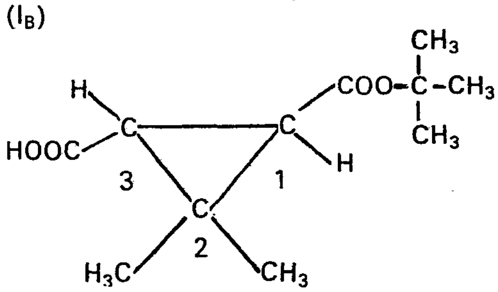

($I_B$)

respectively of (1R,3R) trans or (1S,3S) trans configuration.

2. Process according to Claim 1, characterized in that

– the esterfication agent by the intermediary of which the compound (II) is transformed into its tert-butyl ester is isobutene utilized in the presence of an acid, preferably sulphuric acid, in an organic solvent, preferably methylene chloride:

– the selective hydrolysis of the methyl ester function of the compound ($III_A$) or ($III_B$) is carried out by a mineral base, preferably potassium carbonate, in a water-alcohol solvent, preferably water-methanol;

– the strong base utilized in the isomerization is an alkaline alcoholate, such as sodium methylate, sodium ethylate, or potassium tert-butylate, and the operation is done in an alcohol.

3. Process according to Claim 2, characterized in that the strong base utilized in the isomerization is an alkaline alcoholate, such as sodium methylate, sodium ethylate or potassium tert-butylate, and the operation is done in an alcohol or an ether.

4. Process according to Claim 1, for preparing mono tert-butyl esters of (1R,3S) and (1S,3R) cis 2,2-dimethyl-cyclopropane-1,3-dicarboxylic acids, characterized in that a compound with the formula (II):

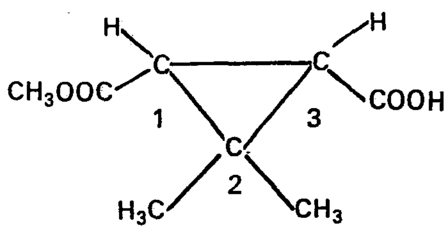

(II)

of (1R,3S) cis or (1S,3R) cis configuration, is made to react with an esterification agent able to transform the compound (II) into its tert-butyl ester, in order to obtain respectively the compound with the formula ($III_A$):

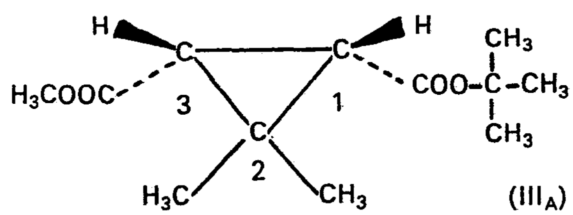

($III_A$)

of (1S,3R) configuration, or the compound with the formula ($III_B$):

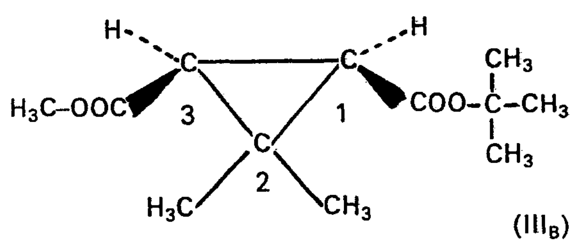

($III_B$)

of (1R,3S) configuration, of which the methyl ester function is selectively hydrolyzed, in order to obtain a compound with the formula ($I_A$):

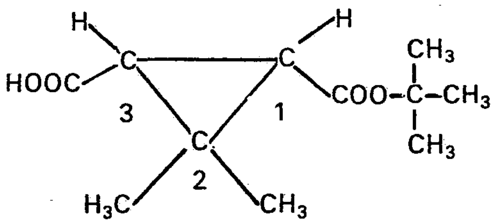

($I_A$)

respectively of (1S,3R) cis or (1R,3S) cis configuration, that is, inverse of that of the compound with the formula (II) used at the start.

5. Process according to Claim 1, for the preparation of the mono tert-butyl esters of (1R,3R) and (1S,3S) trans 2,2-dimethylcyclopropane-1,3-dicarboxylic acids, characterized in that a compound with the formula (II):

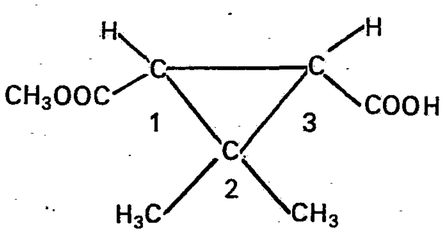

(II)

of (1R,3S) cis or (1S,3R) cis configuration, is made to react with an esterification agent able to transform the compound (II) into its tert-butyl ester, in order to obtain respectively the compound with the formula ($III_A$):

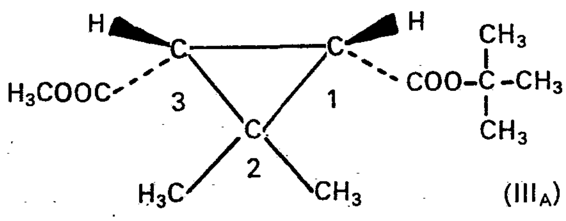

($III_A$)

of (1S,3R) configuration, or the compound with the formula ($III_B$):

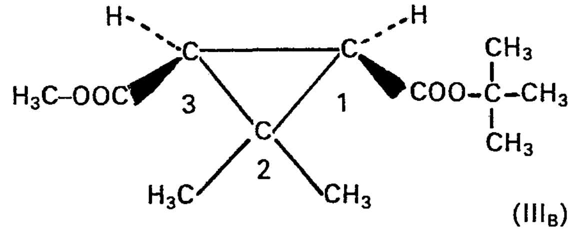

($III_B$)

of (1R,3S) configuration, of which the methyl ester function is selctively hydrolyzed, in order to obtain a compound with the formula ($I_A$)

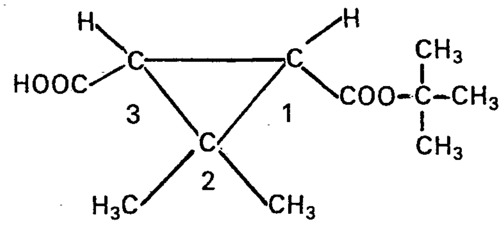

($I_A$)

respectively of (1S,3R) cis or (1R,3S) cis configuration, that is to say, inverse of that of the compound with the formula (II) used at the start, which is isomerized by the action of a strong base, so as to obtain a compound with the formula ($I_B$):

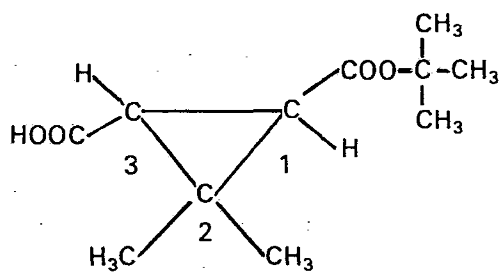

($I_B$)

respectively of (1R,3R) trans or (1S,3S) trans configuration.

Single sheet

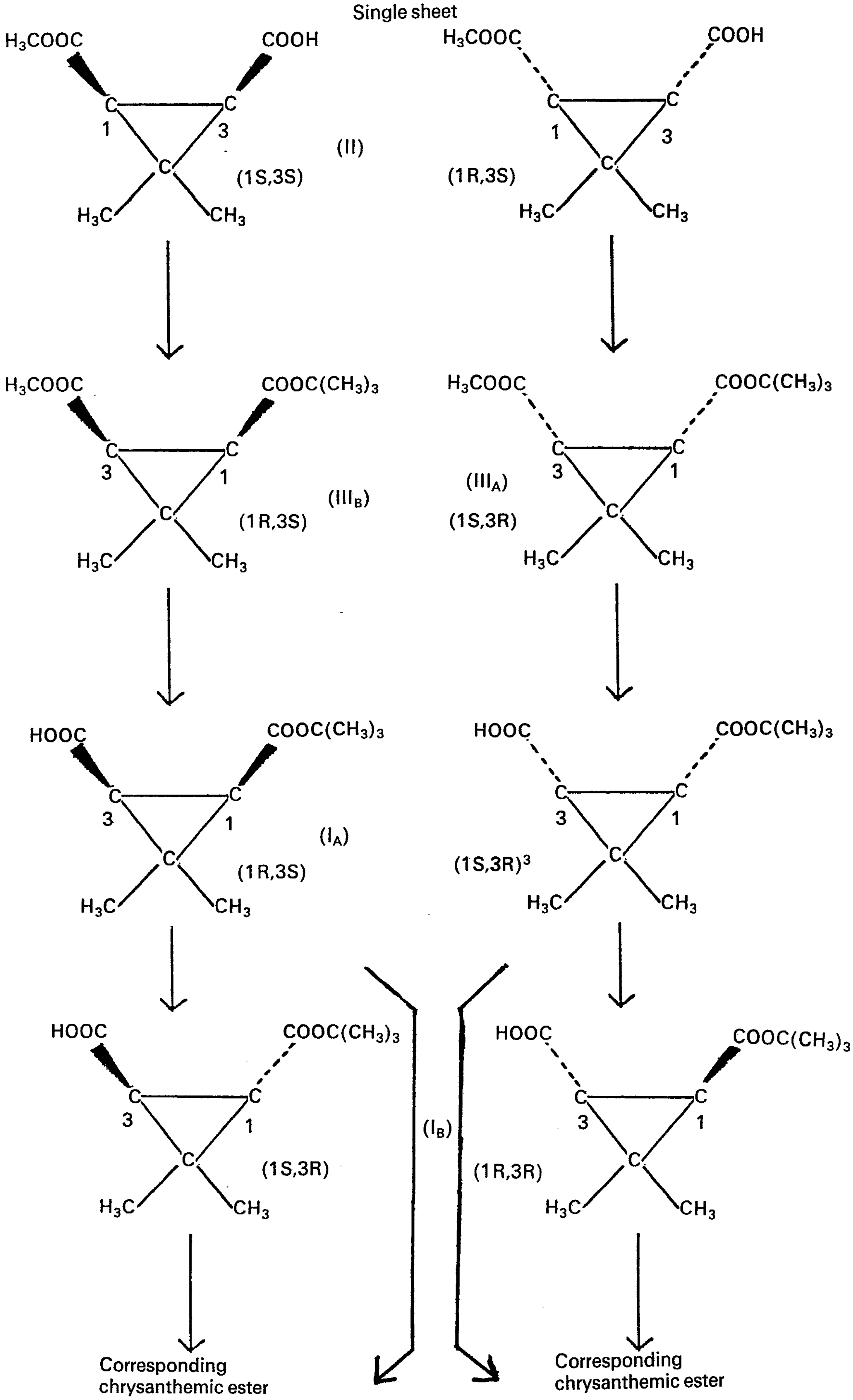


(II)

(1S,3S)

(1R,3S)

($III_B$)

(1R,3S)

($III_A$)

(1S,3R)

($I_A$)

(1R,3S)

(1S,3R)$^{3}$

(1S,3R)

($I_B$)

(1R,3R)